# EUROPÄISCHE PATENTSCHRIFT

(11) **EP 2 965 706 B1**
(45) Veröffentlichungstag und Bekanntmachung des Hinweises auf die Patenterteilung: **23.08.2017**
(21) Anmeldenummer: 15176723.3
(22) Anmeldetag: 31.08.2009
(51) Int. Cl.: A61B 18/20, A61F 9/01, B23K 26/40

(54) **MULTIFUNKTIONALES LASERGERÄT**
MULTIFUNCTIONAL LASER DEVICE
DISPOSITIF LASER MULTIFONCTION

(30) Priorität: 29.08.2008 DE 102008044977
(43) Veröffentlichungstag der Anmeldung: 13.01.2016
(62) Teilanmeldung aus: 09740048.5
(73) Patentinhaber: Starmedtec GmbH, 82319 Starnberg (DE)
(72) Erfinder: Falkenstein, Werner, 82319 Starnberg (DE); Schubert, Michael, 82327 Tutzing (DE); Weidemann, Gregor, 81479 München (DE)
(74) Vertreter: Pfenning, Meinig & Partner mbB

(56) Entgegenhaltungen:
- WO-A1-99/07438
- WO-A1-2008/072033
- WO-A2-2006/128038
- US-A1- 2004 220 456
- US-A1- 2008 086 118
- US-E1- R E37 585

## Beschreibung

Die Erfindung bezieht sich auf ein multifunktionales Lasergerät der im Oberbegriff des Anspruchs 1 genannten Art.

Auf dem Gebiet der Materialbearbeitung allgemein werden in zunehmendem Umfang Laser eingesetzt, die Material abtragen. Hierbei wird typisch mit Lichtimpulsen gearbeitet, die aufgrund der hierbei eingesetzten Festkörper-Laser eine niedrige Impulswiederholfrequenz, beispielsweise im Bereich von 1 bis 500Hz, jedoch Energien aufweisen, die weit über den Schwellen für einen Abtrag liegen, um einen ausreichend schnellen Abtrag zu erzielen.

Lasergeräte verschiedenster Art werden auch im Bereich der Medizin, beispielsweise auf dem Gebiet der Urologie, der Gastroenterologie, der HNO wie auch anderer medizinischer Disziplinen, eingesetzt, um Obstruktionen bzw. ganz generell Gewebe oder Konkremente aller Art zu entfernen, da sie den Abfluss von Körperflüssigkeiten be- oder verhindern oder anderweitig die Gesundheit oder das Wohlbefinden stören und so zu erheblichen gesundheitlichen Komplikationen führen.

Beispielsweise werden in der Urologie obstruierende Harnsteine in den Nieren, den Harnleitern oder der Harnblase zertrümmert, aber auch obstruierende Gewebeveränderungen wie Neoplasien, Tumore oder Vernarbungen, und gutartige Prostatavergrößerungen entfernt.

Analog treten in der Gastroenterologie Konkremente wie auch Weichgewebeobstruktionen in den galleabführenden Wegen auf, von der Leber, in der die Gallenflüssigkeit erzeugt wird, über die Gallengänge, die Gallenblase bis zum Eintritt des Gallengangs in den Zwölffingerdarm. Ähnliches gilt beim Pankreas mit Pankreassteinen. In der HNO treten Obstruktionen in den Speicheldrüsen und -gängen auf, in der Augenheilkunde in den unteren Tränenwegen vom Auge bis in den Nasenbereich.

Es wurde zunächst versucht, Dauerstrichlaser (Nd:YAG) für die Lithotripsie einzusetzen. Dies führte jedoch zu einer langsamen Aufheizung des Steines, bis auf Temperaturen von über 1000°C, wobei der Stein zerfiel, jedoch große Schädigungen des umgebenden Gewebes auf Grund der hohen und lang anhaltenden erzeugten Temperatur auftraten.

Auch Diodenlaser im Bereich von 800 - 1000 nm erweisen sich hierfür als ungeeignet.

Es wurde weiterhin festgestellt, dass Nd:YAG-Laser wie auch Diodenlaser im Wellenlängenbereich von 940 nm - 1540 nm im CW-Betrieb beim Abtrag von Gewebe nicht kontrollierbare tiefe Nekrosezonen bei geringem Abtrag setzen, einhergehend mit starker Karbonisation. Während erstere vermieden werden sollten, da hierbei benachbarte Organe in Mitleidenschaft gezogen werden können, sind letztere als solche nicht unbedingt schädlich für die Gesundheit des Patienten. In der Energiebilanz ist dies jedoch ein Prozess mit hohem Energieverbrauch und damit im Hinblick auf einen effizienten Abtragungsprozess nachteilig. Entsprechend wird der Nd:YAG-Laser in der Regel nicht mehr eingesetzt.

Stattdessen werden im medizinischen Bereich derzeit hauptsächlich gepulste Holmiumlaser mit einer Wellenlänge von 2080 nm eingesetzt, um harte Konkremente zu zertrümmern oder auch Hart- und Weichgewebe zu verdampfen. Hierzu werden Impulsenergien von 200 mJ bis zu 4200 mJ eingesetzt, die Wiederholraten sind im Bereich von 3 Hz bis zu 50 Hz, die Impulsdauern liegen im Bereich von 150 µs - 700 µs.

Die Impulsspitzenleistung eines einzelnen Laserimpulses eines typischen Holmiumlasers beträgt bis zu 15 kW. Diese wird neben der Wellenlänge als bestimmend für die Wechselwirkung des Laserlichtes mit dem Stein bzw. dem Gewebe angesehen. Die Wirkungsweise ist, gleich ob Stein oder Gewebe, weitgehend vergleichbar: das im Stein/Gewebe befindliche Wasser dient als hauptsächlicher Absorber der Wellenlänge des Holmiumlasers mit einem hohen Absorptionskoeffizienten von 28 cm⁻¹, es wird bei Applikationen der verwendeten Laserimpulse mit den genannten Impulsdauern schlagartig auf eine Temperatur von mehreren 100°C gebracht, die über dem regulären Siedepunkt von Wasser (100°C) liegt (überhitztes Wasser). Dieses Wasser verwandelt sich in hoch komprimierten Wasserdampf, der, da noch auf das kleine Bestrahlungsvolumen beschränkt, extrem schnell expandiert und so Gewebe- wie Steinpartikel mit sich reisst. Dieser Abtragprozess ist deshalb so interessant, da nur ein Bruchteil, Abschätzungen ergeben ca. 30% des bestrahlten Volumens mit dem dafür notwendigen Energieaufwand in Dampf umgewandelt werden muss, der Rest wird durch den rasch expandierenden Wasserdampf mechanisch mitgerissen. Dieser Prozess funktioniert nur dann optimal, wenn die Bedingungen des "Heat confinement" gegeben sind, das heißt die Impulsdauer der applizierten Strahlung deutlich kürzer ist, als die für das Material charakteristische Zeit für die Wärmeleitung (thermische Relaxationszeit). Ist diese Bedingung erfüllt, dient im Wesentlichen die gesamte applizierte Energie dem Abtrag, während nur ein unwesentlicher Bruchteil innerhalb dieser Zeit in benachbarte Gewebeareale abfließen kann. Der Abtrag erfolgt entsprechend weitgehend athermisch, der erzeugte Krater im Gewebe wird nur von einem dünnen Saum von koaguliertem Gewebe umgeben, der zwar häufig ausreicht, kleinere Blutgefäße zu versiegeln, aber dennoch nicht zu einer völlig blutungsfreien Behandlung führt. Weiterhin ist die Größe der erzeugten Krater abhängig von der Impulsenergie, je größer diese ist, umso größer auch Kratertiefe und -durchmesser. Es können so einzelne Krater aneinander gereiht werden, ähnlich den Stichen einer Nähmaschine, aber kein glatter, feiner Schnitt, wie z.B. beim Einsatz eines Skalpells erreicht werden.

Damit sind sind Holmiumlaser dazu geeignet, sowohl Steine zu zertrümmern als auch Gewebe abzutragen bzw. dieses zu schneiden, dieses allerdings nur in vergleichsweise grober Weise.

Während Holmiumlaser für die reine Steinbehandlung mit Leistungen von 10-20 W, Energien von 0.2-2J und Wiederholraten von 3-10 Hz als Desktopgeräte gebaut werden können, mit Abmessungen von ca. 50x50x30 cm³ und einem Gewicht von 30-40 kg, sind für höhere Leistungsklassen von 60-100 W mit Energien von 0,2-4,2J und Wiederholraten von bis zu 50 Hz große Standgeräte erforderlich, die Abmessungen von 50x110x80 cm³ aufweisen, und bis zu 160 kg schwer sind. Diese großen, schweren und auch teuren Geräte sind erforderlich, um die für einen ausreichenden Gewebeabtrag oft für erforderlich gehaltenen hohen Wiederholraten von 30-50 Hz zu erzeugen. Typisch werden mehrere Laserstrahlquellen zeitlich versetzt jeweils mit geringerer Frequenz betrieben, um so die erforderliche externe hohe Wiederholrate zu erzeugen, z.B. 4 Laserstrahlquellen mit jeweils 10 Hz und 20 W zur Erzeugung von 40 Hz, 80 W.

Derzeitige Holmiumlaser werden daher gegenwärtig vorzugsweise für die Lithotripsie verwendet und weisen Fragmentationsraten von 0,2 mg/Impuls bei einer Energie von 1000 mJ auf. Dies führt zu Fragmentationsraten von 120 mg/Minute bei 1000 mJ/10 Hz und hochgerechnet 240 mg/min bei 2000 mJ/10 Hz. An diesen Fragmentationsraten müssen sich zukünftige Lithotripsiegeräte messen lassen.

Weiterhin werden neben den Holmiumlasern Dauerstrich- (CW-) Laser im Wellenlängenbereich von 1,8 - 2,1 µm eingesetzt, die Gewebe fein und präzise schneiden und auch verdampfen, die aber in der Regel Steine nicht zertrümmern können, so dass ihr Einsatzbereich beschränkt ist. Typische derzeitige 2 µm CW-Laser sind:
- Dioden gepumpte Thuliumlaser mit einer festen Wellenlänge von 2010 nm
- Dioden gepumpte Faserlaser mit festen Wellenlängen im Bereich von 1900 - 2000 nm.
- Laserdioden im Bereich von 1800 - 2100 nm auf Basis von (AlGaln)(GaSb).

Gegenwärtig im Einsatz befindliche Thulium- und Faserlaser können zwar getaktet werden, hierdurch ändert sich aber die Spitzenleistung nicht: ein z.B. bei 100 W betriebener Laser kann extern moduliert werden, z.B. eine Impulsdauer von 10 ms eingestellt werden, in gleichem Maß reduziert sich aber auch die Energie auf 1 J, so dass sich wiederum eine Impulsspitzenleistung von 100 W ergibt, bei einer Wiederholrate von z.B. 10 Hz die abgegebene Leistung von 100 W auf 10 W sinkt.

Für einen präzisen chirurgischen Effekt mit kontrollierbaren geringen bzw. in ihrer Größe gewünschten Nebeneffekten (z.B. Koagulationszone) müssen die Parameter der Energiequelle (hier: Lasergerät) geeignet ausgewählt werden. Um überhaupt einen chirurgischen Effekt zu erzielen, müssen in dem zu bearbeitenden Material Absorber vorliegen, die die Wellenlänge des applizierten Lichtes absorbieren und so den gewünschten Effekt erzeugen. Im menschlichen Körper liegen unterschiedliche Absorber vor, deren wichtigste Wasser, Blut und Melanin sind.

Entsprechend der Höhe der Absorption bei der eingestrahlten Laserwellenlänge und der Konzentration des Absorbers ergeben sich Eindringtiefe und (chirurgische) Effekte. Besonders geeignet erscheinen Absorptionsspitzen (z.B. Blut bei 540 nm bzw. 580 nm) und die verschiedenen Wasserabsorptionsspitzen im mittleren Infrarotbereich (1,5 / 2 / 3 µm), um eine möglichst selektive Wirkung unter weitestgehender Schonung benachbarter oder darunterliegender Gewebe zu erzeugen. Die Absorptionskoeffizienten und damit die Eindringtiefen variieren hierbei erheblich. Die Eindringtiefe sollte hierbei so gewählt werden, dass sie der Größe der Zielstruktur angepasst ist. Soll z.B. die Epidermis mit einer Dicke von ca. 100 µm abgetragen werden, erscheint der Erbiumlaser mit einer Wellenlänge von 2,94 µm am besten geeignet, da er das Gewebe in Schichten von einigen 10 µm abzutragen gestattet und somit in mehreren Durchgängen die Epidermis gezielt abgetragen werden kann.

Demgegenüber eignet sich die Absorptionsspitze bei 2 µm mit einer Eindringtiefe der Laserstrahlung von etwa 0,1 bis 0,5 mm (entspricht einem Absorptionskoeffizienten von 100 cm⁻¹ bis 20 cm⁻¹) gut für chirurgische Anwendungen allgemeiner Art, bei der die Größe der Zielstrukturen im Bereich von einigen Millimetern bzw. bis zu Zentimetern (beim Schneiden) liegt.

Bisheriger Stand der Technik ist es, zwei Laser unterschiedlicher Wellenlänge (1,06 µm und 1,32 µm oder 980 nm und 2,0 µm) zu verwenden, um unterschiedliche Licht-Gewebe-Wechselwirkungen zu erzeugen.

Aus der EP-B-1079897 ist es weiterhin bekannt, bei unveränderter Wellenlänge (z.B. 2,94 µm) durch Modulation der Emission eines Er-Lasers in gewissen Grenzen von rein abtragenden Impulsen auf Impulse mit einer gewissen begrenzten oberflächlichen thermischen Wirkung (Koagulationszonen 100 µm) zu wechseln. Diese Koagulationszone ist z.B. ausreichend, um feinste kapilläre Blutungen der oberen Dermis zu verschließen, aber nicht, um größere Blutgefäße sicher zu koagulieren. Die Änderung der Licht-Gewebe-Wechselwirkung ist hierbei also recht begrenzt.

Für einen effizienten Abtrag ist es aber auch erforderlich, dass die applizierte Energie weitestgehend für den Abtrag zur Verfügung steht und nicht durch konkurrierende Prozesse, wie Wärmeleitung und Strukturänderung verloren geht. Um die Bedingungen des "thermal confinement" - "thermischen Einschlusses" zu erfüllen, muss die Impulsdauer deutlich kürzer sein als die thermische Relaxationszeit, die bei biologischem Material im Wesentlichen durch den Wasserabsorptionskoeffizienten gegeben ist. Für eine Impulsdauer, die einen Faktor >2 unter dieser rechnerischen Relaxationszeit liegt, ist zum Ende des Laserimpulses die gesamte applizierte Energie im bestrahlten Volumen konzentriert, während bei Impulsdauern, die einen Faktor >2 über dieser rechnerischen Größe liegen, sich die absorbierte Energie zum Ende des Laserimpulses über ein größeres Volumen verteilt und sich so die Spitzentemperatur im bestrahlten Volumen beträchtlich reduziert hat.

Es ist zu errechnen, dass für den Bereich von 1920 nm-1940 nm von Wasser die thermische Relaxationszeit etwa 8 ms beträgt und demzufolge die Bedingungen des thermischen Einschlusses bei Impulsdauern unter 4 ms gegeben sind. Dies bedeutet konkret, dass für einen effizienten Abtrag, gleichgültig ob Stein oder Gewebe, Impulsdauern von <4 ms zu bevorzugen sind.

Da in der Regel nicht nur das im Gewebe befindliche Wasser das eingestrahlte Licht absorbiert, sondern auch das Gewebe selbst, ergibt sich eine weitere Grenze: die Absorption der Laserimpulse führt unmittelbar nach dem eigentlichen Absorptionsprozess zu mechanischen Ausdehnungseffekten, die die eingestrahlte Energie abführen, noch bevor die Effekte der Wärmeleitung einsetzen. Bei diesen deutlich kürzeren Einstrahldauern kann es zu sogenannten Stresswellen führen, und gelingt es, den Effekt der Stresswellen auf das bestrahlte Volumen zu begrenzen, führt dies zu maximalen Zug- und Druckwellen, die den Gewebeverbund mechanisch aufreißen. Dies kann wiederum zu einer nochmaligen Erhöhung der Abtragseffizienz von Gewebe führen. Typisch sind, abhängig von der Gewebestruktur, Impulsdauern von fs bis ns erforderlich, um diese Bedingung zu erfüllen.

Da Gewebe typischerweise aus unterschiedlich großen Strukturen aufgebaut sind (Ausnahme: Cornea), können nur generelle Aussagen getroffen werden, in der Art, dass eine Verkürzung der Impulsdauern vom Millisekundenbereich über Mikrosekunden bis in den.Nano- und Pikosekundenbereich Verbesserungen in der Ablationseffizienz erwarten lassen, diese ist für jeden Gewebetyp und Wellenlänge in Abhängigkeit von der Impulsdauer getrennt in vivo zu ermitteln.

Aufgrund der Weiterentwicklung von Diodenlasern in Richtung auf eine höhere Leistung und einen höheren Gesamt-Wirkungsgrad werden zunehmend auch Diodenlaser eingesetzt, da sie als Tischgeräte konzipiert werden können. Wesentlich ist hierbei zusätzlich, dass nicht nur die nominale, vom Lasergerät abgegebene Leistung relevant ist, sondern die im Gewebe tatsächlich zur Verfügung stehende Leistung betrachtet werden muss. Diese ergibt sich aus der Geräteleistung nach Abzug von Oberflächenreflektion und Rückstreuung. Letztere beträgt ca. 55% bei 1064 nm, 25% bei 940-980 nm, aber nur 5% bei 2 µm Wellenlänge, was wiederum bedeutet, dass der mittlere Infrarotbereich von 1,4 bis 3,5 µm zu bevorzugen ist.

Derzeit stehen Laserdioden auf der Basis von (AlGaln)(GaSb) oder AlGaln)(InP) zur Verfügung, die teilweise direkt im Wellenlängenbereich von 1800-2300 nm abstrahlen. Diese Laserdioden werden als reine Dauerstrichlaser mit Leistungen von bis zu 18 W angeboten. Höhere Leistungen sind erst in einigen Jahren zu erwarten.

Aus der DE 199 12 992 ist ein Verfahren zur punktuellen oder flächigen Lichtbestrahlung unter Verwendung von Laserdioden bekannt, bei dem die Impulsleistung und/oder die Impulsdauer und/oder die Wiederholfrequenz bei weitgehend konstanter mittlerer Leistung vom Benutzer oder automatisch in Abhängigkeit von der angestrebten Wirkung geändert werden können, wobei eine Anzahl von Laserdioden gleichzeitig oder alternierend verwendet wird. Die hierbei verwendeten Leistungen liegen unterhalb der Abtragschwelle. Hierbei ist es das Ziel, eine therapeutische Wirkung durch Ändern elektrochemischer Bedingungen von Molekülen in Zellen herbeizuführen. Weiterhin wird hierbei eine einzige feste Wellenlänge verwendet, die durch die Art der Laserdiode oder Laserdioden festgelegt ist.

Die US 2008/0086118 A1 beshreibt einen diodengepumpten Festkörperlaser (diode-pumped solid-state laser - DPSSL) und die Anwendung laserinduzierter thermisch explosiver Ablation in der Chirurgie der Prostata. Es wird ein quasi-CW Q-geschalteter DPSSL bei einer geringeren Wiederholungsrate von 5 kHz bis 20 kHz betrieben, um eine größerer Pulsenergie von 2.5 mJ bis 10 mJ zu produzieren, während eine optimale mittlere Leistung aufrechterhalten wird. Außerdem werden zwei AO Q-Schalter oder ein EO Q-Schalter verwendet, um kürzere Impulsdauern von 40 ns bis 150 ns und eine höhere Spitzenleistung zwischen 50 kW bis 100 kW zu erhalten, was zu einer einer Pulsfluenz von 250 mJ/cm² bis 1000 mJ/cm² auf Zielgewebe führt. Außerdem wird eine elektronischen Steuerung verwendet, mit der eine Amplitudenfluktuation unter 5% unterdrückt wird, um ein glatteres Ablationsprofil sicherzustellen.

Die WO 2008/072033 A1 beschreibt eine chirurgische Vorrichtung und ein Verfahren zur Behandlung von biologischen harten Geweben, basierend auf einer faseroptischen Wellenleiterstruktur, welche umfasst: eine Quelle von kohärenter Lichtstrahlung mit einem Faserabschnitt, der mit einem Element der Metalle der Seltenen Erden, insbesondere Thulium, dotiert ist, um einen aktiven Bereich zu bilden, der angeordnet ist, Photonen bei optischen Wellenlängen in dem Bereich zwischen 1400 nm and 2400 nm, und vorzugsweise bei der Wellenlänge von 1940 nm, zu emittieren, und einen faseroptischen Wellenleiterabschnitt, der sich über den aktiven Bereich hinaus erstreckt, um die Lichtstrahlung auf die Oberfläche des zu behandelnden Gewebes zu lenken.

Aus dem Vorstehenden ist zu erkennen, dass bisher für die jeweiligen Anwendungszwecke unterschiedliche oder aufwändige Lasergeräte erforderlich waren, die einen relativ großen Raum- und Leistungsbedarf haben und kostspielig sind.

Der Erfindung liegt die Aufgabe zu Grunde, ein multifunktionales Lasergerät zu schaffen, das bei einfachem und raumsparenden Aufbau und geringem Leistungsbedarf einen weiten Anwendungsbereich ergibt.

Diese Aufgabe wird ausgehend von einem multifunktionalen Lasergerät zur Bearbeitung von Material, insbesondere biologischem Material, wobei das von einer Laserlichtquelle abgegebene Licht eine Wellenlänge aufweist, bei der das Material einen hohen (> 10 cm⁻¹) Absorptionskoeffizienten aufweist und der Laser Lichtimpulse mit einer jeweiligen Impulsdauer und Impulswiederholfrequenz liefert, erfindungsgemäß dadurch gelöst, dass die Lichtimpulse eine Energie, die zwischen dem Einfachen und dem Zwanzigfachen der Schwellenenergie des Materials für einen Abtrag liegt, und eine Wiederholfrequenz im Bereich mehr als 20 kHz aufweisen.

Gemäß einer Ausgestaltung der Erfindung ist die Laserlichtquelle eine aus einer steuerbaren Stromversorgung gespeiste Laserdiode, wobei die Stromversorgung mit einer Steuereinrichtung zur Ansteuerung der Laserdiode mit veränderbarem Strom und/oder veränderbarer Impulsdauer und/oder Impulswiederholfrequenz versehen ist.

Hierbei kann der Laserdioden-Strom während der Impulse mit gegenüber dem CW-Betrieb um einen Faktor von mehr als 2 erhöhtem Strom betrieben werden.

Vorzugsweise kann der Laserdioden-Strom während der Impulse in einer Impulswiederholzeit bei Impulsdauern von 1 bis 50 µs um den Faktor 5 bis 50 gegenüber dem Nennstrom der Laserdiode vergrößert sein.

Bei einer anderen Ausgestaltung der Erfindung ist vorgesehen, dass die Impulsdauer der Lichtimpulse der Laserdiode im Piko- bis Nanosekunden-Bereich liegt.

Dabei kann der Laserdioden-Strom während der Impulse in einer Impulswiederholzeit bei Impulsdauern von 10-1000 ns um den Faktor 10 bis 100 gegenüber dem Nennstrom der Laserdiode (1) vergrößert sein.

Bei Impulsdauern der Impulse in einer Impulswiederholzeit von 10ps-10 ns kann der Laserdioden-Strom um den Faktor 50 bis 1000 gegenüber dem Nennstrom der Laserdiode (1) vergrößert sein.

Weiterhin ist vorzugsweise vorgesehen, dass Wellenlänge des Lasergerätes schnell änderbar ist und dass die Steuereinrichtung zur Steuerung des Stromes an die mindestens eine Laserdiode (1) derart ausgebildet ist, dass sich eine schnelle Änderung der Wellenlänge über einen Betriebs-Wellenlängenbereich ergibt, der die Wellenlänge zumindest eines Bereichs mit einer wesentlichen Änderung des Absorptionskoeffizienten (Absorptionsspitze, starker Anstieg oder Abfall der Absorption) des Materials einschließt.

Die Änderung der Wellenlänge kann hierbei über eine Zeit im Bereich von weniger als einer Sekunde erfolgen.

Die Laserdiode kann bei einer weiteren Ausgestaltung der Erfindung die Pumplichtquelle eines zweiten Lasers bilden, dessen Ausgangslicht das Ausgangslicht der Laserlichtquelle bildet.

Der zweite Laser kann hierbei ein Festkörperlaser oder ein Faserlaser sein.

Gemäß einer weiteren Ausgestaltung der Erfindung ist die Laselichtquelle ein hochfrequent laufender Faserlaser mit einer Wiederholfrequenz von 20 bis 100kHz und Impulsdauern von 10 bis 50µs.

Gemäß einer bevorzugten Ausgestaltung der Erfindung ist vorgesehen, dass eine Einstelleinrichtung zur Veränderung der Energie der Lichtimpulse und deren Wellenlänge in Abhängigkeit von dem Ausgangssignal eines Detektionssystems ausgebildet ist, das zur Erkennung von Konkrementen, Blutgefäßen, Nerven, Grenzschichten (Tumor, Prostatakapsel o.a.) über eine Erkennung der Autofluoreszenz von Gewebe, Detektion einer Fluoreszenz nach Anregung über zweite Lichtquelle und/oder in Kombination mit OCT ausgebildet ist.

Häufig wird der Wellenlängenbereich von 1800-2100 nm einfach nur als der Bereich eines der Absorptionspeaks von Wasser angesehen, ohne auf die detaillierte Struktur des Absorptionskoeffizienten von Wasser und dessen Auswirkungen auf die Licht-Material-Wechselwirkung zu achten. Die Absorptionsspitze weist von 1,92-1,94 µm hohe Werte von 120-130 cm⁻¹ auf. Der gesamte Bereich erscheint gleichermaßen geeignet zu sein. Ein hoher Absorptionskoeffizient bedeutet eine geringe Abtragsschwelle und damit einen effizienten Abtrag bei gegebener Energie bzw. Leistung.

Erfindungsgemäß wird aufbauend auf dem höheren Absorptionskoeffizienten und damit geringeren Abtragsschwellen mit vergleichsweise geringen Impulsenergien (1 bis 50 mJ), aber mit entsprechend höheren Wiederholfrequenzen (1 bis 100 kHz), ein suffizienter Abtrag auch an harten Steinen erzielt. Nimmt man an, dass sich in diesem begrenzten Bereich von 1,8 bis 2,3 µm Absorptionskoeffizient und Abtragsschwelle umgekehrt proportional verhalten, lässt dies, aufbauend auf Literaturwerten bei 2080 nm, eine Abtragsschwelle von ca. 1,3 mJ erwarten, ein Wert, der sich mit zur Verfügung stehenden Geräten in der Leistungsklasse von 50 W und einer Impulsdauer von 20 µs erzielen lässt.

Die in der Literatur angegebenen Werte für den Absorptionskoeffizienten werden in der Regel für Zimmertemperatur bestimmt (20 °C). Es wurde jedoch festgestellt, dass der Absorptionskoeffizient von Wasser Temperatur abhängig ist und die Absorptionskurve bei höheren Temperaturen leicht ansteigt und sich zu kürzeren Wellenlängen hin verschiebt. Da der Abtrag von Stein oder Gewebe in der Regel bei Temperaturen um den Siedepunkt erfolgt, ist es sehr viel realistischer, den Absorptionskoeffizienten bei 80°C als Grundlage für die Optimierung von Gerätespezifikationen heranzuziehen. Will man sich die Vorteile eines möglichst hohen Absorptionskoeffizienten zu Nutze machen, ergibt sich hieraus, dass unter Berücksichtigung des Temperatur abhängigen Absorptionskoeffizienten von Wasser eine Wellenlänge von 1,92 µm anderen Wellenlängen vorzuziehen ist, da dieser bei einer Temperaturerhöhung von 20 °C auf 80°C sogar leicht von 128 1/cm auf 145 1/ cm anwächst, während z.B. 1,94 µm - bei 20°C weitgehend gleich zu 1,92 µm, demgegenüber deutlich von 124 1/cm auf 120 1/cm abfällt.

Durch die erfindungsgemäße Ausgestaltung des multifunktionalen Lasergerätes ist ein weiter Anwendungsbereich bei geringer Leistungsaufnahme und damit Raum und Gewicht sparendem Aufbau möglich.

Damit kann ein kostengünstiges Gerät mit kleinen Abmessungen als Tischgerät mit optimierten Eigenschaften nicht nur für das präzise Schneiden (CW-Betrieb) sondern auch für die Entfernung bzw. Zerkleinerung von Obstruktionen aller Art - Steine, verschiedene Gewebearten, mit kleinen Abmessungen und geringem Gewicht, günstigen Herstellkosten, intraoperativ schnell veränderlicher Wellenlänge und/oder Impulsdauer geschaffen werden.

Eine Lithotripsie kann weiterhin mit geringen Energien, kurzen Impulsdauer, aber hohen Repetitionsraten, ohne zusätzlichen Aufwand für einen Güteschalter durchgeführt werden, ohne dass die Gefahr eines Festklebens einer Faserspitze am Gewebe oder einer extremen Überhitzung und eines Abbrennens der Faserspitze besteht, da bei gepulstem/getaktetem Betrieb die Pausen lang genug gewählt werden können, um diese hohen Temperaturen an der Faserspitze sicher zu vermeiden.

Während Festkörperlaser im Bereich des nahen und mittleren Infrarotbereichs wie der Neodym, der Holmium- oder der Thuliumlaser physikbedingt, aber auch Faserlaser auf Grund des die Wellenlänge bestimmenden Bragg-Gitters am Faserende in ihrer Wellenlänge im Wesentlichen festgelegt sind, weisen Diodenlaser typischerweise eine gewisse, vom jeweiligen System abhängige Temperaturverschiebung der Wellenlänge auf. In der Regel wird versucht, diese zu minimieren, sei es durch eine spezifische Auslegung der Abfolge von Schichten im Aufbau des Diodensystems, sei es durch starke thermische Anbindung an (Mikrokanal-) Kühler und deren aktive oder passive Kühlung. Natürlich kann damit über z.B. eine externe Kühlmitteltemperatur bzw. das Maß des Luftdurchsatzes bei Luftkühlung auch in gewissen Grenzen die Emissionswellenlänge auf einen gewünschten Sollwert eingestellt werden. Dieser Prozess ist aber langsam (typischerweise mehr als eine Minute).

Ein Diodensystem mit einer vergleichsweise größeren Temperaturabhängigkeit kann demgegenüber über einen weiteren Wellenlängenbereich eingestellt werden und so die Lichtquelle über einen größeren Bereich einer Absorptionskurve verschoben werden, sodass die sonst unerwünschte Temperaturabhängigkeit in vorteilhafter Weise zur Erzielung der gewünschten Wirkung ausgenutzt werden kann.

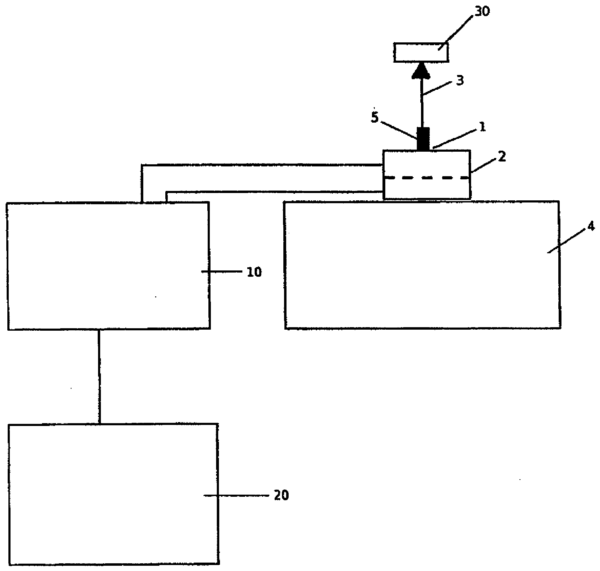

Die Erfindung wird weiter anhand einer in der Zeichnung gezeigten schematischen Darstellung eines Lasergerätes näher erläutert.

In der Zeichnung ist eine Ausführungsform eines Lasergerätes gezeigt, das zumindest eine auf einem Kühlelement 4 in wärmeleitender Verbindung mit diesem angeordnete Laserdiode 1 mit einer Grenzschicht 2 aufweist.

Das Kühlelement 4 kann eine steuerbare Kühlleistung aufweisen.

Das Ausgangslicht der zumindest einen Laserdiode 1 wird über ein Auskoppelelement 5 in eine Lichtleitfaser 3 eingekoppelt, deren freies Ende auf ein zu bearbeitendes Material 30 gerichtet oder mit diesem in Kontakt gebracht wird.

Es ist jedoch auch möglich, das Ausgangslicht des oder der Laserdioden 1 zunächst als Pumplicht in einen Festkörper- oder Faserlaser einzukoppeln, dessen Ausgangslicht in die Lichtleitfaser eingekoppelt wird.

Die Laserdiode 1 wird aus einer Stromversorgung 20 über eine Steuereinrichtung 10 mit einem Strom gespeist, der durch die Steuereinrichtung in vielfältiger steuer- und programmierbarer Weise änderbar ist.

Eine schnelle Veränderung der Wellenlänge kann sehr einfach bewerkstelligt werden, wenn berücksichtigt wird, dass die Lumineszenzwellenlänge von derartigen Laserdiodensystemen, das heißt die Wellenlänge bei geringer Leistung und die Arbeitswellenlänge auf Grund der Temperaturabhängigkeit des jeweiligen Materialsystems deutlich unterschieden sind. So differiert die Lumineszenzwellenlänge von (AlGaln)(GaSb)-Laserdioden im Wellenlängenbereich von 2000 nm um bis zu 55 nm von der Arbeitswellenlänge, die bei Maximalstrom und damit Maximalleistung definiert ist. So ergaben eigene Messungen an einem Diodenmodul mit nominal 13 W Maximalleistung folgende Werte:
70 A / 13 W/1940 nm
30 A/ 5W/1905nm
15 A/2,3W/1885nm

Bei einer Wellenlänge von 1920 nm bei Maximalleistung ist demzufolge eine Wellenlänge von 1865 nm für eine Leistung von 2,3 W zu erwarten. Der Absorptionskoeffizient von Wasser ändert sich hierbei von 128 cm⁻¹ auf etwa 17 cm⁻¹.

Die Wellenlänge der Diode 1 folgt ohne wesentliche Zeitverzögerung der Temperatur der Grenzschicht für den Laserübergang, so dass über eine schnelle Änderung des Diodenstroms die Wellenlänge gleichermaßen schnell verändert werden kann, allerdings unter Veränderung der abgegebenen optischen Leistung. Dies kann jedoch durchaus erwünscht sein, wenn z.B. zunächst mit einer Wellenlänge gearbeitet wird, bei der eine hohe Materialabsorption vorliegt, dann aber bei Treffen auf ein anderes Material 30 mit anderen Eigenschaften mit geänderter Wellenlänge und vielleicht sogar verminderter Leistung gearbeitet werden soll.

Die Lumineszenzwellenlänge liegt aber nicht nur bei ganz geringen Leistungen vor, sondern auch bei sehr kurzen Impulsdauern deutlich unter ca. 1 µs, ein Wert oberhalb dem für die Laserdiode im Wesentlichen der CW-Betriebsbereich vorliegt. Soll jedoch z.B. die abgegebene optische Leistung konstant gehalten und nur die Wellenlänge geändert werden, kann dies in der Form geschehen, dass nicht mit maximaler Leistung gearbeitet wird, aber mit Impulsen unterschiedlicher Impulsdauer und Wiederholfrequenz: z.B. für Wellenlänge 1 mit Impulsen im Quasi-CW-Betrieb mit Impulsen >>1 µs, z.B. 20 µs, aber geringer Wiederholfrequenz und für die Wellenlänge 2 mit kurzen Impulsen <<1 µs z.B. 200 ns, aber höherer Wiederholfrequenz.

Damit kann nun ein Lasersystem in einfacher und gegebenenfalls auch schneller Weise (<1 sec) in diesem Bereich nicht nur hinsichtlich seiner abgegebenen Energie, sondern auch hinsichtlich seiner Wellenlänge verändert werden, so dass die Wechselwirkung Licht-Material (Wasser/Gewebe) in noch größerem Maße verändert werden und so besser an den vorgesehenen Anwendungszweck angepasst werden kann. So kann von hoher Absorption und damit geringerer Eindringtiefe gut für nebenwirkungsarmes präzises Schneiden von Gewebe, schnell auf verringerte Absorption mit größerer Eindringtiefe und damit verstärkten thermischen Wirkungen z.B. zur verbesserten Blutstillung größerer Gefäße gewechselt werden.

Derzeit stehen Laserdioden auf der Basis von (AlGaln)(GaSb) oder (AlGaln)(InP) zur Verfügung, die teilweise direkt im Wellenlängenbereich 1800-2300 nm abstrahlen. Diese Laserdioden werden als reine Dauerstrichlaser mit Leistungen von bis zu 18 W angeboten.

Diese Diodenlaser sind derzeit in Abhängigkeit vom Wellenlängenbereich in unterschiedlichen Leistungsvarianten erhältlich:
940-980 nm: Leistungen bis 150 W / 200 W
1800-2200 nm: Leistungen bis zu 18 W

Es ist bekannt, dass Diodenlaser mit geeigneten Ansteuerungen ohne zusätzliche externe Güteschalter gepulst betrieben werden können. Da die Diodenfacetten in der Regel nur bestimmte Maximalleistungen aushalten, ergibt sich hier nur eine mäßige typische Impulsüberhöhung von 20-50% (CW: 120 W, getaktet im Bereich von Impulsdauern von mehr als 10 ms:150 W).

Es wurde jedoch erfindungsgemäß festgestellt, dass beispielsweise Laserdioden auf der Basis (AlGaln)(GaSb) im Impulsdauerbereich von einigen ps über 100 ns bis ca. 100 µs um erhebliche Faktoren (derzeit Faktor 50 bei 50 ps bis Faktor 10 bei 10 µs) überpulst werden können, ohne dass es zu einer Schädigung der Facetten bzw. zu einem irreversiblen thermischen roll-over kommt, das heißt gegenüber der CW-Leistung steigt die Impulsspitzenleistung um einen entsprechenden Faktor an. Damit können Leistungseinbußen, wie sie bei der Taktung von z.B. Dioden gepumpten Faserlasern oder Dioden-gepumpten Festkörperlasern zwangsläufig sind, zumindest teilweise kompensiert werden.

Bei geeigneter Optimierung für den Betrieb mit Überpulsung gegenüber dem CW-Betrieb sind weitere Verbesserungen im Hinblick auf höhere Leistung der Einzelemitter und das Maß der Überpulsbarkeit zu erwarten.

Die Impulsdauer kann so leicht in einen Bereich verlegt werden, bei dem die Bedingungen des heat-confinement oder sogar des stress-confinement vorliegen, und ein entsprechender hocheffizienter Abtrag erzielt werden.

Gegenüber bisherigen Annahmen konnte nachgewiesen werden, dass ein ausreichender Abtrag bei deutlich geringeren Einzeilmpulsenergien von 10-50 mJ erzielbar ist, dies dann mit einer deutlich höheren Wiederholrate von 1-100 kHz. Die Kühlung muss nur auf die effektive mittlere Leistung ausgelegt sein, durch geeignete Zusatzschaltungen lässt sich die Energie und Leistung eines für den CW-Betrieb ausgelegten Netzteils speichern und in kurzen Entladungen mit entsprechend überhöhten Strömen, ähnlich wie bei Blitzlampen-gepumpten Holmiumlasern abrufen.

Erfindungsgemäß wird aufbauend auf dem höheren Absorptionskoeffizienten und damit geringeren Abtragsschwellen mit vergleichsweise geringen Impulsenergien, aber mit entsprechend höheren Frequenzen, ein suffizienter Abtrag auch an harten Steinen erzielt. Nimmt man an, dass sich in diesem begrenzten Bereich von 1,8 bis 2,3 µm Absorptionskoeffizient und Abtragsschwelle umgekehrt proportional verhalten, lässt dies eine Abtragsschwelle von ca. 1,3 mJ erwarten, ein Wert, der sich mit zur Verfügung stehenden Geräten in der Leistungsklasse von 50 W und einer Impulsdauer von 20 µs unter Einsatz der Überpulsung erzielen lässt.

Unter Einsatz eines CW-Faserlasers durchgeführte Untersuchungen ergeben Folgendes:
Abtragsschwelle bei 1,92 µm und 230 µm Faser: 0,15 mJ/2000 Hz; 0,23 mJ/1000 Hz; 0,32 mJ/500 Hz;
Abtragsschwelle bei 1,92 µm und 365 µm Faser:0,25 mJ/2000 Hz; 0,51 mJ/1000 Hz; 1,02 mJ/500 Hz;
(Einbeziehung der Fläche: Erhöhung um Faktor 2,5; Experiment: im Mittel 2,357).

Hieraus kann geschlossen werden, dass die Abtragsschwelle im Bereich von 0,2-1,3 mJ zu liegen kommt.

In praxi wird zur Erzielung eines guten Abtrags deutlich über der Ablationsschwelle gearbeitet. Verwendet man ein 50 W-Gerät mit einem Duty cycle von 50%, also effektiv 25 W und 500 Hz, so ergibt sich eine Einzelimpulsenergie von 50 mJ mit einer Impulsdauer von 1 ms (Faktor 250-400 über der Abtragsschwelle).

Bei 500 Hz/25 W konnte so an einem natürlichen KalziumOxalatmonohydratstein (sehr harter Stein, eine Fragmentationsrate von 52 mg/min und damit eine Verdoppelung der Abtragseffizienz gegenüber Literaturwerten erzielt werden.

Eine Vergleichsmessung mit einem Dioden gepumpten Thulium CW-Laser (Wellenlänge von 2,01 µm, Absorptionskoeffizient in Wasser: 64 cm⁻¹ ergab mit einer 230 µm Faser und einer Laserleistung von 20,5 W an einem Kunststein einen Abtrag von 8,6 mg/min (Totalfragmentation), während bei einer Wellenlänge von 1,92 µm und 25 W ein Abtrag von 45,8 mg/min zu erzielen war, immerhin um einen Faktor 4 höher als bei 2,01 µm. Ein Faktor 2 lässt sich auf den um Faktor 2 geringeren Absorptionskoeffizienten der TM-Wellenlänge von 2,01 µm zurückführen, der verbleibende Faktor 2,5 der verkürzten Impulsdauer zuzuordnen.

Die aufgeführten Messungen beziehen sich auf Dioden gepumpte Faserlaser ohne jede Impulsüberhöhung. Bei Dioden auf Basis (AlGalN)(GaSb) konnte gezeigt werden, dass Überpulsungen um erhebliche Faktoren erzielt werden können, ohne diese zu zerstören. Damit ergibt sich unter Einsatz multipler Emitter (Barren von z.B. 19 Emittern), Kopplung mehrerer solcher Barren, wie auch bei entsprechend hoher Überpulsung bei Einzelemittern die Möglichkeit, die durch eine Verkürzung der Impulsdauer bedingte Energiereduktion gegebenenfalls vollständig zu kompensieren, mit dem zusätzlichen Effekt, dass die Abtragungseffizienz nochmals ansteigt.

Ähnlich kann in einfacher Weise das Licht von 3 Barren in eine Faser eingekoppelt werden. Unter Verwendung unterschiedlicher Polarisationen kann das Licht von 6 Barren in Fasern eingekoppelt werden und so Energie und Leistung nach oben skaliert werden. Bei entsprechend kurzen Impulsdauern von Pikosekunden können Überpulsbarkeiten um den Faktor 1000 und mehr erwartet werden.

Beispiele für den Steinabtrag mit Diodenlasern, wie sie im Vergleich zu einem Ho:YAG-Laser ermittelt wurden, sind in der nachfolgenden Tabelle angegeben.

| Laser | Energie mJ | Impulsdauer µs | Imp. Spritzenleistung W | Wiederholfrequenz Hz | Mittl. Leistung W | Überpulsung Faktor |
|---|---|---|---|---|---|---|
| Ho:YAG | 400 | 200 | 2000 | 15 | 6 | NA |
| Diode 20 E | 20 | 10 | 2000 | 300 | 6 | 10 |
| Diode 20 E | 2 | 1 | 2000 | 3000 | 6 | 50 |
| Diode EE | 2 | 0,5 | 4000 | 3000 | 6 | 100 |
| Diode EE | 1 | 0,5 | 2000 | 6000 | 6 | 50 |

Diode EE bedeutet hierbei Werte für Einzelemitter, Diode 20 E die Verwendung eines Barrens mit 20 Einzelemittern. Ähnlich kann in einfacher Weise das Licht von 3 Barren in eine Faser eingekoppelt werden, unter Verwendung unterschiedlicher Polarisationen kann das Licht von 6 Barren in Fasern eingekoppelt werden und so Energie und Leistung nach oben skaliert werden. Bei entsprechend kurzen Impulsdauern von Pikosekunden können Überpulsbarkeiten um den Faktor 1000 und mehr erwartet werden. Die hierbei verwendeten Wellenlängen lagen im Bereich von 1,92 µm bis 1,94 µm.

In gleicher Weise können Festkörper- und Faserlaser mit überpulsbaren Dioden gepumpt werden und so die Impulsspitzenleistung gegenüber dem CW-Betrieb deutlich erhöht werden.

Weiterhin enthält diese Anmeldung die folgenden Aspekte:
1. Multifunktionales Lasergerät zur Bearbeitung von Material, insbesondere biologischem Material (30), wobei das von einer Laserlichtquelle (1) abgegebene Licht eine Wellenlänge aufweist, bei der das Material einen hohen (> 10 cm⁻¹) Absorptionskoeffizienten aufweist und der Laser Lichtimpulse mit einer jeweiligen Impulsdauer und Impulswiederholfrequenz liefert,
   dadurch gekennzeichnet, dass die Lichtimpulse eine Energie, die zwischen dem Einfachen und dem Zwanzigfachen der Schwellenenergie des Materials für einen Abtrag liegt, undeineWiederholfrequenz im Bereich mehr als 300 Hz aufweisen.
2. Lasergerät nach Aspekt 1, dadurch gekennzeichnet, dass der Absorptionskoeffizient bei der Wellenlänge größer als 10 cm⁻¹ ist.
3. Lasergerät nach Aspekt 1 oder 2, dadurch gekennzeichnet, dass die Wiederholfrequenz größer als 3 kHz ist.
4. Lasergerät nach Aspekt 1 oder 2, dadurch gekennzeichnet, dass die Wiederholfrequenz größer als 6 kHz ist.
5. Lasergerät nach Aspekt 1 oder 2, dadurch gekennzeichnet, dass die Wiederholfrequenz größer als 20 kHz ist.
6. Lasergerät nach einem der Aspekte 1 bis 5, dadurch gekennzeichnet, dass die Laserlichtquelle eine aus einer steuerbaren Stromversorgung (10, 20) gespeiste Laserdiode (1) ist, wobei die Stromversorgung mit einer Steuereinrichtung (10) zur Ansteuerung der Laserdiode (1) mit veränderbarem Strom und/oder veränderbarer Impulsdauer und/oder Impulswiederholfrequenz versehen ist.
7. Lasergerät nach Aspekt 6, dadurch gekennzeichnet, dass der Laserdioden-Strom während der Impulse in einer Impulswiederholzeit bei Impulsdauern von 1-100 µs um den Faktor 5 bis 50 gegenüber dem Nennstrom der Laserdiode vergrößert ist.
8. Lasergerät nach Aspekt 6, dadurch gekennzeichnet, dass die Impulsdauer der Lichtimpulse der Laserdiode (1) im Piko- bis Nanosekunden-Bereich liegt.
9. Lasergerät nach Aspekt 8, dadurch gekennzeichnet, dass der Laserdioden-Strom während der Impulse in einer Impulswiederholzeit bei Impulsdauern von 10-1000 ns um den Faktor 10 bis 100 gegenüber dem Nennstrom der Laserdiode (1) vergrößert ist.
10. Lasergerät nach Aspekt 8, dadurch gekennzeichnet, dass der Laserdioden-Strom während der Impulse in einer Impulswiederholzeit bei Impulsdauern von 10ps-10 ns um den Faktor 50 bis 1000 gegenüber dem Nennstrom der Laserdiode (1) vergrößert ist.
11. Lasergerät nach einem der Aspekte 6 bis 10, dadurch gekennzeichnet, dass die Laserlicht-Wellenlänge des Lasergerätes schnell änderbar ist und dass die Steuereinrichtung (10) zur Steuerung des Stromes an die mindestens eine Laserdiode (1) derart ausgebildet ist, dass sich eine schnelle Änderung der Wellenlänge über einen Betriebs-Wellenlängenberei ch ergibt, der die Wellenlänge zumindest eines Bereichs mit einer wesentlichen Änderung des Absorptionskoeffizienten des Materials einschließt.
12. Lasergerät nach Aspekt 11, dadurch gekennzeichnet, dass die Steuereinrichtung (10) für eine schnelle Änderung der Wellenlänge über eine Zeit im Bereich von weniger als einer Sekunde ausgebildet ist.
13. Lasergerät nach einem der Aspekte 6 bis 12, dadurch gekennzeichnet, dass die Laserdiode (1) die Pumplichtquelle eines zweiten Lasers bildet, dessen Ausgangslicht das Ausgangslicht der Laserlichtquelle bildet.
14. Lasergerät nach Aspekt 13, dadurch gekennzeichnet, dass die der zweite Laser ein Festkörperlaser ist.
15. Lasergerät nach Aspekt 13, dadurch gekennzeichnet, dassdiederzweite Laserein Faserlaser ist.
16. Lasergerät nach Aspekt 1, dadurch gekennzeichnet, dass die. Laserlichtquelle ein hochfrequent laufender Faserlaser mit einer Wiederholfrequenz von 20 bis 100kHz und Impulsdauern von 10 bis 50µs ist.
17. Lasergerät nach einem der vorhergehenden Aspekte, dadurch gekennzeichnet, dass eine Einstelleinrichtung zur Veränderung der Energie der Lichtimpulse und deren Wellenlänge in Abhängigkeit von dem Ausgangssignal eines Detektionssystems ausgebildet ist, das zur Erkennung von Konkrementen, Blutgefäßen, Nerven, Grenzschichten (Tumor, Prostatakapsel o.a.) über eine Erkennung der Autofluoreszenz von Gewebe, Detektion einer Fluoreszenz nach Anregung über zweite Lichtquelle und/oder in Kombination mit OCT ausgebildet ist.
18. Lasergerät nach einem der vorhergehenden Aspekte, dadurch gekennzeichnet, dass das Material biologisches Gewebe ist.
19. Lasergerät nach Aspekt 18, dadurch gekennzeichnet, dass der in dem Gewebe enthaltene Absorber Wasser ist.
20. Lasergerät nach Aspekt 18 oder 19, dadurch gekennzeichnet, dass die Wellenlänge des Laserlichts im mittleren IR-Bereich von 1,4 bis 3,3 µm liegt.
21. Lasergerät nach Aspekt 18 oder 19, dadurch gekennzeichnet, dass die Wellenlänge des Laserlichts bei 1,92 µm liegt.

## Patentansprüche

1. Multifunktionales Lasergerät zur Bearbeitung von Material, insbesondere biologischem Material (30), wobei das von einer Laserlichtquelle (1) des Lasergeräts abgegebene Licht eine Wellenlänge aufweist, bei der das Material einen Absorptionskoeffizienten von mehr als 10 cm⁻¹ aufweist und der Laser Lichtimpulse mit einer jeweiligen Impulsdauer und Impulswiederholfrequenz liefert,
**dadurch gekennzeichnet, dass**
die Lichtimpulse eine Energie, aufweisen, die zwischen dem Einfachen und dem Zwanzigfachen der Schwellenenergie des Materials für einen Abtrag liegt, wobei die Impulsenergie zwischen 1 und 50 mJ liegt, die Impulswiederholfrequenz zwischen 20 und 100 kHz liegt und die Wellenlänge des Laserlichts im mittleren IR-Bereich von 1,4 bis 3,3 µm liegt.

2. Lasergerät nach Anspruch 1, **dadurch gekennzeichnet, dass** die Laserlichtquelle mindestens eine aus einer steuerbaren Stromversorgung (10, 20) des Lasergeräts gespeiste Laserdiode (1) umfasst, wobei die Stromversorgung mit einer Steuereinrichtung (10) zur Ansteuerung der Laserdiode (1) mit veränderbarem Strom und/oder veränderbarer Impulsdauer und/oder Impulswiederholfrequenz versehen ist.

3. Lasergerät nach Anspruch 2, **dadurch gekennzeichnet, dass** der Laserdioden-Strom während der Impulse in einer Impulswiederholzeit bei Impulsdauern von 1-100 µs um den Faktor 5 bis 50 gegenüber dem Nennstrom der Laserdiode vergrößert ist.

4. Lasergerät nach Anspruch 2, **dadurch gekennzeichnet, dass** die Impulsdauer der Lichtimpulse der Laserdiode (1) im Piko- bis Nanosekunden-Bereich liegt.

5. Lasergerät nach Anspruch 4, **dadurch gekennzeichnet, dass** der Laserdioden-Strom während der Impulse in einer Impulswiederholzeit bei Impulsdauern von 10-1000 ns um den Faktor 10 bis 100 gegenüber dem Nennstrom der Laserdiode (1) vergrößert ist.

6. Lasergerät nach Anspruch 4, **dadurch gekennzeichnet, dass** der Laserdioden-Strom während der Impulse in einer Impulswiederholzeit bei Impulsdauern von 10 ps bis 10 ns um den Faktor 50 bis 1000 gegenüber dem Nennstrom der Laserdiode (1) vergrößert ist.

7. Lasergerät nach einem der Ansprüche 2 bis 6, **dadurch gekennzeichnet, dass** die Laserlicht-Wellenlänge des Lasergerätes schnell änderbar ist, dass die Steuereinrichtung (10) zur Steuerung des Stromes an die mindestens eine Laserdiode (1) derart ausgebildet ist, dass sich eine schnelle Änderung der Wellenlänge über einen Betriebs-WellenlängenBereich ergibt, der die Wellenlänge zumindest eines Bereichs mit einer wesentlichen Änderung des Absorptionskoeffizienten des Materials einschließt, und dass die Steuereinrichtung (10) für eine Änderung der Wellenlänge über eine Zeit im Bereich von weniger als einer Sekunde ausgebildet ist.

8. Lasergerät nach einem der vorangehenden Ansprüche, **dadurch gekennzeichnet, dass** ein zweiter Laser vorgesehen ist, dessen Pumplichtquelle die Laserdiode (1) bildet, und dessen Ausgangslicht das Ausgangslicht der Laserlichtquelle bildet.

9. Lasergerät nach Anspruch 8, **dadurch gekennzeichnet, dass** der zweite Laser ein Festkörperlaser oder Faserlaser ist.

10. Lasergerät nach Anspruch 1, **dadurch gekennzeichnet, dass** die Laserlichtquelle ein hochfrequent laufender Faserlaser mit einer Wiederholfrequenz von 20 bis 100 kHz und Impulsdauern von 10 bis 50 µs ist.

11. Lasergerät nach einem der vorhergehenden Ansprüche, **dadurch gekennzeichnet, dass** eine Einstelleinrichtung zur Veränderung der Energie der Lichtimpulse und deren Wellenlänge in Abhängigkeit von dem Ausgangssignal eines Detektionssystems des Lasergeräts ausgebildet ist, das zur Erkennung von Konkrementen, Blutgefäßen, Nerven, Grenzschichten (Tumor, Prostatakapsel, o. a.) über eine Erkennung der Autofluoreszenz von Gewebe, Detektion einer Fluoreszenz nach Anregung über eine zweite Lichtquelle und/oder in Kombination mit OCT ausgebildet ist.

12. Lasergerät nach einem der vorangehenden Ansprüchen, **dadurch gekennzeichnet, dass** die Wellenlänge des Laserlichts bei 1,92 µm liegt.

## Claims

1. Multifunctional laser device for treating material, in particular biological material (30), the light emitted by a laser light source (1) of the laser device having a wavelength at which the material has an absorption coefficient of more than 10 cm⁻¹ and the laser light source supplying light pulses with a respective pulse duration and pulse repetition frequency,
**characterised in that** the light pulses have an energy which is between one and twenty times the threshold energy of the material for a removal, wherein the energy of the pulses is between 1 and 50 mJ, the pulse repetition frequency is between 20 and 100 kHz and the wavelength of the laser light is in the mid-IR range of 1.4 to 3.3 µm.

2. Laser device according to claim 1, **characterised in that** the laser light source comprises at least one laser diode (1) fed from a controllable current supply (10, 20), the current supply being provided with a control device (10) to drive the laser diode (1) with variable current and/or variable pulse duration and/or pulse repetition frequency.

3. Laser device according to Claim 2, **characterised in that** the laser diode current is increased, during the pulses in a pulse repetition time at pulse durations of 1-100 µs, by a factor of 5 to 50 compared with the nominal current of the laser diode.

4. Laser device according to Claim 2, **characterised in that** the pulse duration of the light pulses of the laser diode (1) is in the picosecond to nanosecond range.

5. Laser device according to Claim 4, **characterised in that** the laser diode current is increased during the pulses in a pulse repetition time at pulse durations of 10-1000 ns, by a factor of 10 to 100 compared with the nominal current of the laser diode (1).

6. Laser device according to Claim 4, **characterised in that** the laser diode current is increased, during the pulses in a pulse repetition time at pulse durations of 10 ps-10 ns, by a factor of 50 to 1000 compared with the nominal current of the laser diode (1).

7. Laser device according to one of Claims 2 to 6, **characterised in that** the wavelength of the laser light of the laser device enables a quick change of the wavelength, that the control device (10) to control the current to the at least one laser diode (1) is configured in such a manner that there results a quick change of the wavelength over an operating wavelength range which includes the wavelength of at least one range with a substantial change of the absorption coefficient of the material, and **in that** the control device (10) is configured for a change of the wavelength over a time in the range of less than one second.

8. Laser device according to one of the preceding Claims, **characterised in that** a second laser is provided, the pump light of which forms the laser diode and the output light of which forms the output light of the laser light source.

9. Laser device according to claim 8, **characterised in that** the second laser is a solid-state laser or fibre laser.

10. Laser device according to Claim 1, **characterised in that** the laser light source is a high-frequency-operating fibre laser with a repetition frequency of 20 to 100 kHz and pulse durations of 10 to 50 µs.

11. Laser device according to one of the preceding claims, **characterised in that** an adjusting device for varying the energy of the light pulses and their wavelength in dependence on the output signal of a detection system which is configured to detect concretions, blood vessels, nerves, boundary layers (tumour, prostatic capsule or the like) by a detection of the autofluorescence of tissue, detection of fluorescence after excitation via a second light source and/or in combination with OCT.

12. Laser device according to one of the preceding claims, **characterised in that** the wavelength of the laser light is 1.92 µm.

## Revendications

1. Dispositif laser multifonctionnel pour le traitement d'une matière, en particulier d'une matière biologique (30), la lumière émise par une source lumineuse laser (1) du dispositif laser ayant une longueur d'onde pour laquelle la matière a un coefficient d'absorption de plus de 10 cm⁻¹ et la source lumineuse laser fournissant des impulsions lumineuses avec une durée d'impulsion et une fréquence de répétition d'impulsions respectives,
**caractérisé en ce que** les impulsions lumineuses ont une énergie qui se situe entre une fois et vingt fois l'énergie seuil de la matière pour un enlèvement de matière, où l'énergie des impulsions et comprise entre 1 et 50 mJ, la fréquence de répétition des impulsions est comprise entre 20 et 100 kHz et la longueur d'onde de la lumière laser se situe dans la plage médiane de la lumière infrarouge de 1,4 à 3,3 µm.

2. Dispositif laser selon la revendication 1, **caractérisé en ce que** la source lumineuse laser comprend au moins une diode laser (1) alimentée par une alimentation en courant (10, 20) apte à être commandée, l'alimentation en courant étant pourvue d'un dispositif de commande (10) pour commander ladite diode laser (1) avec un courant variable et/ou une durée d'impulsions variable et/ou une fréquence de répétition des impulsions.

3. Dispositif laser selon la revendication 2, **caractérisé en ce que** le courant des diodes laser est agrandi 5 à 50 fois par rapport au courant nominal de la diode laser pendant les impulsions durant un temps de répétition avec des durées d'impulsions de 1 à 100 µs.

4. Dispositif laser selon la revendication 2, **caractérisé en ce que** la durée d'impulsion des impulsions lumineuses de la diode laser (1) se situe dans la plage des picosecondes à nanosecondes.

5. Dispositif laser selon la revendication 4, **caractérisé en ce que** le courant des diodes laser est agrandi 10 à 100 fois par rapport au courant nominal de la diode laser (1) pendant les impulsions durant un temps de répétition avec des durées d'impulsions de 10 à 1000 ns.

6. Dispositif laser selon la revendication 4, **caractérisé en ce que** le courant des diodes laser est agrandi 50 à 1000 fois par rapport au courant nominal de la diode laser (1) pendant les impulsions durant un temps de répétition avec des durées d'impulsions de 10 ps à 10 ns.

7. Dispositif laser selon l'une quelconque des revendication 2 à 6, **caractérisé en ce que** la longueur d'onde de la lumière laser du dispositif laser permet une variation rapide de la longueur d'onde, **en ce que** ledit dispositif de commande (10) pour la commande du courant sur ladite au moins une diode laser (1) est adapté de manière à obtenir une variation rapide de la longueur d'onde sur une plage de longueur d'onde de fonctionnement qui inclut la longueur d'onde d'au moins une zone avec variation sensible du coefficient d'absorption de la matière, et **en ce que** le dispositif de commande (10) est adapté pour une variation de la longueur d'onde pendant une durée dans la plage inférieure à une seconde.

8. Dispositif laser selon l'une quelconque des revendications précédentes, **caractérisé en ce qu'**il est prévu un deuxième laser dont la lumière de pompage forme la diode laser et dont la lumière de sortie forme la lumière de sortie de la source lumineuse laser.

9. Dispositif laser selon la revendication 8, **caractérisé en ce que** le deuxième laser est un laser à solide ou un laser à fibre.

10. Dispositif laser selon la revendication 1, **caractérisé en ce que** la source lumineuse laser est un laser à fibre fonctionnant à haute fréquence avec une fréquence de répétition de 20 à 100 kHz et des durées d'impulsions de 10 à 50 µs.

11. Dispositif laser selon l'une quelconque des revendications précédentes, **caractérisé en ce qu'**un dispositif de réglage pour faire varier l'énergie des impulsions lumineuses et leur longueur d'onde en fonction du signal de sortie d'un système de détection, est adapté pour détecter des concrétions, des vaisseaux sanguins, des nerfs, des couches limites (tumeur, capsule prostatique, etc.) par l'intermédiaire d'une détection de l'autofluorescence des tissus, détection d'une fluorescence après stimulation via une deuxième source lumineuse et/ou une combinaison avec la TCO.

12. Dispositif laser selon l'une quelconque des revendications précédentes, **caractérisé en ce que** la longueur d'onde de la lumière laser se situe autour de 1,92 µm.
